# EUROPEAN PATENT SPECIFICATION

(11) **EP 4 096 525 B1**
(45) Date of publication and mention of the grant of the patent: **07.08.2024**
(21) Application number: 21704705.9
(22) Date of filing: 29.01.2021
(51) Int. Cl.: A61B 5/20, A61B 5/00, E03D 11/00

(54) **A TOILET SYSTEM WITH SENSORS FOR MEASURING EXCRETION OUTPUT OF A USER, A FLUID BALANCE MONITORING SYSTEM AND A METHOD FOR DETERMINING AND MONITORING THE FLUID BALANCE OF A SUBJECT**
TOILETTENSYSTEM MIT SENSOREN ZUM MESSEN DER AUSSCHEIDUNGSLEISTUNG EINES BENUTZERS, EIN FLÜSSIGKEITSBILANZ-ÜBERWACHUNGSSYSTEM UND EIN VERFAHREN ZUM BESTIMMEN UND ÜBERWACHEN DER FLUIDBILANZ EINES PROBANDEN
SYSTÈME DE TOILETTES DOTÉ DE CAPTEURS PERMETTANT DE MESURER LA SORTIE D'EXCRÉTION D'UN UTILISATEUR, SYSTÈME DE SURVEILLANCE D'ÉQUILIBRE FLUIDIQUE ET PROCÉDÉ PERMETTANT DE DÉTERMINER ET DE SURVEILLER L'ÉQUILIBRE FLUIDIQUE D'UN SUJET

(30) Priority: 31.01.2020 DK PA202070063
(43) Date of publication of application: 07.12.2022
(60) Divisional application: 24165211.4
(73) Proprietor: Measurelet ApS, 2970 Hørsholm (DK)
(72) Inventor: BAGGER, Marie Lommer, 2830 Virum (DK); BO SØNDERGAARD SVENDSEN, Morten, 2800 Kongens Lyngby (DK)
(74) Representative: AWA Denmark A/S
(86) International application number: PCT/DK2021/050029
(87) International publication number: WO 2021/151446

(56) References cited:
- JP-A- 2007 270 584
- JP-A- 2018 109 285
- US-A1- 2018 087 969
- US-B1- 9 743 903

## Description

### Technical Field

The present invention relates to a toilet system comprising a bowl and an outlet, the toilet system being adapted for discerning between types of excretion excreted into the bowl by a user of the toilet system. The present invention further relates to a method for determining a type of excretion excreted into a bowl of a toilet system according to any one of the above claims.

As used herein, the term "excretion", when used as a noun, is intended to encompass in principle any waste product that is eliminated from a human body, but particularly urine and faeces. Likewise, as used herein the term "excretion" and conjugations thereof, when used as a verb, is intended to encompass the action of expelling in principle any waste product that is to be eliminated from a human body.

As used herein, the term "main axis" is intended to refer to an axis parallel with any one of a base, particularly a base line, and a principal axis, particularly a minor axis, of an object, depending on the specific geometrical shape of the object.

As used herein, the term "liquid pressed through a water seal of the toilet system", and in particular "pressed through", is intended to encompass liquid pressed through a water seal of the toilet system irrespective of how, i.e. including not only by an excretion forcing liquid through the water seal, but also overflowing given that it is the force of gravity pressing on the liquid.

As used herein the terms "subject" and "mammalian subject" are intended to encompass in principle any subject for which it is desired to monitor the fluid balance, but particularly such subjects being relevant for the health sector, i.e. mammals such as particularly human beings.

### Background Art

Knowledge of the type and quantity of excretion excreted by patients is of great importance within the health sector since healthcare professionals, such as nurses and doctors, use this information for diagnostic purposes and for monitoring the progress of diseases and convalescence in patients. For instance, this information may be of great value in determining the correct treatment for a patient and whether the treatment chosen is effective or needs adjustment.

Toilets adapted for measuring faecal and urinal output of a user are commonly known. However, by far most known solutions are limited to addressing the issue of measuring the quantity of urine excreted into a toilet by a user. This has in the prior art been done both using weight sensors and using pressure sensors.

Within the health sector, particularly on hospitals, it is therefore furthermore known as common practice for healthcare professionals to measure the quantity of faeces excreted into a toilet or a bedpan by a patient by physically collecting and weighing the faeces. This process is, however, extremely time consuming and cumbersome, and furthermore impractical from a hygiene-related perspective, for users and healthcare professionals alike. Also, the use of bedpans may be unappealing or even humiliating to the patient or user.

Furthermore, US 2018/368818 A1 discloses a toilet device which includes a toilet body that includes a toilet bowl part, a camera that photographs faeces discharged and falling into the toilet bowl part in time series and acquires a plurality of still images of the faeces, and a faecal properties estimation part that estimates a change in property of the faeces from the plurality of still images acquired by the camera. The toilet device estimates the change in the property of the faeces from the still images of the faeces photographed in time series by the camera.

US 2018/087969 A1 discloses an in-toilet apparatus for discrimination of urine and faeces, in which an optical sensor is provided for the analysis of urination and defecation events within a toilet bowl. Additionally, strain gauges are provided to make possible the quantification of urine and faeces released by a user. The combination of data from the optical sensors and strain gauges makes it possible for a user to readily know the mass of their bodily excrements, characterized by excrement type. In several embodiments the optical sensor is a thermal camera mounted on the toilet seat or in the toilet bowl. In other embodiments the optical sensor is a water level sensor. In varying embodiments, strain gauges are located in the toilet seat or in an attached foot-scale or both.

US 9 743 903 B1 discloses a toilet system with one or more microphones and a controller used to detected, transmit, and store toilet noise data. One or more speakers are used to provide user feedback based on the detected toilet noise data. Speakers, microphones, and circuitry may be located within a toilet seat of a toilet. A user device or remote device may be connected to the toilet noise detection toilet apparatus for data recording, collection, and health trend reporting.

JP 2007-270584 A discloses a toilet system quantifying excretion excreted into a toilet bowl using an air pressure sensor positioned in a chamber arranged downstream of a water seal between the toilet bowl and an outlet of the toilet system.

JP 2018-109285 A discloses a toilet system measuring a urine volume by measuring liquid flow using a sensor arranged downstream of a water seal between the toilet bowl and an outlet of the toilet system.

The prior art toilets, however, have the drawback that the measurements performed are not sufficiently precise. Furthermore, the prior art toilets are at best not able to properly discern between faecal and urinal matter when excreted into the bowl of the toilet by a user. Still further, the prior art toilets are not able to assess the quantity of faecal matter when excreted into the bowl of the toilet by a user.

It is thus desired to provide a toilet alleviating at least some of the above-mentioned and other drawbacks related to the prior art.

### Summary of Invention

It is therefore the object of the invention to provide a toilet of the type mentioned in the introduction with which the precision of the measurements performed is increased, which is capable of properly discerning between faecal and urinal matter when excreted into the bowl of the toilet by a user, and which is able to accurately assess the quantity of faecal matter when excreted into the bowl of the toilet by a user.

A further object of the invention is to provide such a toilet which is easy and quick to use, and which provides for a more appealing and hygienic solution, for users, patients and healthcare professionals alike.

The invention is defined by the subject matter of the independent claims. Particular embodiments of the invention are set out in the dependent claims.

The above and other objects are in a first aspect of the invention achieved by means of a toilet system comprising a bowl and an outlet, the toilet system being adapted for discerning between types of excretion excreted into the bowl by a user of the toilet system, the toilet system further comprising at least one first sensor device configured to capture at least one signal indicative of a type of excretion excreted into the bowl by a user of the toilet system, and at least one second sensor device configured to capture at least one signal indicative of a quantity of excretion excreted into the bowl by a user of the toilet system, where the second sensor device is arranged in the outlet of the toilet system in a position downstream of a water seal arranged between the bowl and the outlet of the toilet and comprises a chamber adapted for collecting liquid forced through the water seal and a measuring unit arranged and adapted for measuring the quantity of liquid collected in the chamber.

By providing at least one first sensor device configured to capture at least one signal indicative of a type of excretion excreted into the bowl by a user of the toilet system, a toilet system is provided which is capable of properly discerning between faecal and urinal matter when excreted into the bowl of the toilet by a user or patient.

By further providing at least one second sensor device configured to capture at least one signal indicative of a quantity of excretion excreted into the bowl by a user of the toilet system, a toilet system is provided which is also capable of assessing the quantity of faecal matter when excreted into the bowl of the toilet by a user or patient.

As the sensor devices captures the respective signals automatically and without the need of human intervention, a toilet system is provided which is furthermore easy and quick to use, particularly for the user or patient, and which provides for a more appealing and hygienic solution, for users, patients and healthcare professionals alike. Also, time and resources are saved.

Furthermore, such a toilet system provides the data needed by healthcare professionals for diagnostic purposes and for monitoring the progress of diseases and convalescence in patients in a simple, hygienic and reliable manner.

In an embodiment, the at least one first sensor device is configured to measure sounds.

The inventors have found that by measuring the sound related to a user excreting an excretion, it becomes possible with a particularly high degree of certainty and precision to discern between faecal and urinal matter when excreted into the bowl of a toilet.

Furthermore, the inventors have found that by using sound measurements it even becomes possible to also identify the excretion or disposal of other types of matter, such as flatulence or paper, into the bowl of the toilet system from the excretion of faecal and urinal matter, and thereby to remove unwanted and/or irrelevant data such as false positives from the resulting data.

Thereby a toilet system is provided with which the precision of the measurements performed is increased, and which is capable of properly and with a high precision discerning between faecal and urinal matter when excreted into the bowl of the toilet by a patient or user.

A further advantage lies in that such a type of sensor need not come into physical contact with the excretion in order to capture the needed measurements and signals. Thereby, the hygiene and durability of the toilet system is further increased as it is easy to clean and keep clean.

In an embodiment, the at least one first sensor device comprises a vibration sensor, such as an acoustic sensor or a microphone.

Thereby a toilet system is provided which is particularly simple in construction, and which is particularly easy to clean.

In an embodiment, the at least one first sensor device is arranged in or at the toilet seat.

Thereby a toilet system is provided which the captured signals indicative of a type of excretion excreted into the bowl by a user of the toilet system is provided with a high degree of detail. This especially applies to such signals in the form of sound signals, and especially such sound signals produced by the patient or user.

In an embodiment the at least one first sensor device is arranged in or at the bowl above a waterline.

Thereby a toilet system is provided which the capture of signals indicative of a type of excretion excreted into the bowl by a user of the toilet system is optimized and provided with a particularly great detail. The inventors have shown that due to the acoustics prevailing in the bowl above the water line, this especially applies to such signals in the form of sound signals, and especially such sound signals produced by the patient or user and/or such sound signals produced when excretions come into contact with the bowl or materials in the bowl.

In an embodiment, the at least one first sensor device is arranged in or at the bowl below the waterline.

Thereby a toilet system is provided which the capture of signals indicative of a type of excretion excreted into the bowl by a user of the toilet system is optimized and provided with a particularly great detail. The inventors have shown that due to the acoustics prevailing in the bowl below the water line, this especially applies to such signals in the form of sound signals, and especially such sound signals produced by the excretions upon impact with the water in the bowl.

In an embodiment, the at least one first sensor device further comprises any one or more of a pressure sensor, a radar, an image capturing device, a capacitive sensor, and flow rate sensor.

Thereby, further data for determining the type of excretion excreted into a bowl of a toilet system is provided in a particularly simple, straight forward and cost efficient manner. This in turn provides for a determination of the type of excretion being particularly accurate. Capacitive sensors in particular have the further advantage that they can be mounted on the outside of the water ways, water trap and the like of the toilet system, such as even outside the bowl of the toilet system, whereby a further improvement of the hygiene is achieved.

In an embodiment, the at least one first sensor device is configured to transmit the at least one signal to a data analysis device.

In an embodiment, the at least one second sensor device is configured to transmit the at least one signal to a data analysis device.

As the first sensor device and/or the second sensor device thus also transmits the captured signals to the analysis device automatically and without the need of human intervention, a toilet system is provided which is particularly easy and quick to use, particularly for the user or patient, and which provides for a more appealing and hygienic solution, for users, patients and healthcare professionals alike.

Furthermore, such a toilet system provides the data needed by healthcare professionals for diagnostic purposes and for monitoring the progress of diseases and convalescence in patients in a particularly simple, hygienic and reliable manner, saving both time and resources for the involved individuals.

In an embodiment, the at least one second sensor device is configured to measure a volume of liquid indicative of a quantity of excretion excreted into the bowl by a user of the toilet system.

Thereby a toilet system is provided which is able to accurately assess the quantity of faecal or urinal matter when excreted into the bowl of the toilet by a user, and with which the precision and degree of detail of the data needed by healthcare professionals for diagnostic purposes and for monitoring the progress of diseases and convalescence in patients is increased.

According to the invention, the at least one second sensor device is arranged in the outlet of the toilet system in a position downstream of a water seal arranged between the bowl and the outlet of the toilet system.

Such a positioning of the second sensor device is advantageous especially in toilets with a water seal, a water trap or a u-bend in the outlet as the normal water level in the bowl of such toilets is flush with the upper limit defined by the water seal. Therefore, even a small amount of excretion deposited in the bowl will cause the water in the water seal to spill over into the outlet downstream of the water seal, such that the amount of spilled over water may easily be measured by a second sensor device positioned downstream of the water seal.

The at least one second sensor device may be any one or more of a weight sensor, a level sensor and a flow sensor.

Thereby a toilet system is provided which is capable of properly and with a high precision capturing a signal indicative of the quantity of faecal or urinal matter excreted into the bowl of the toilet by a patient or user, and with which the precision of the measurements performed is increased.

In an embodiment, the toilet system further comprises a data analysis device, the data analysis device comprising a data processing unit and a data storage unit, the data processing unit being configured to receive one or more signals transmitted by the at least one first sensor device, and analyse the received one or more signals such as to produce a data output indicative of at least the type of excretion excreted into the bowl by a user of the toilet system.

Thereby a toilet system is provided with which the precision of the measurements performed is increased, and which is capable of automatically and with a high precision discerning between faecal and urinal matter when excreted into the bowl of the toilet by a patient or user.

A further advantage lies in that analysis of the data may thus be performed automatically and without any need for physical contact with the excretion in order to determine type. Thereby, time and costs otherwise spent by healthcare personnel, not only on analysis of the excretion, but also on associated sterilization and cleaning, is eliminated and the hygiene and durability of the toilet system is further increased.

The analysis may be a comparison with existing data, e.g. from a database, and/or a statistical analysis. The analysis may for instance be performed in a neural network, a principal component analysis (PCA), a decision tree or a clustering.

In an embodiment, the data analysis device is further configured to receive one or more signals transmitted by the at least one second sensor device, and analyse, using the data processing unit, the received one or more signals such as to produce a data output indicative of the quantity of excretion excreted into the bowl by a user of the toilet system.

Thereby a toilet system is provided with which the above-mentioned advantages related to the data analysis device are also extended to the determination of the quantity of excretion.

In an embodiment, the data analysis device further comprises a data visualization unit, and the data processing unit is further configured to visualize on the data visualization unit the data output indicative of one or more of the type of excretion excreted into the bowl by a user of the toilet system and the quantity of excretion excreted into the bowl by a user of the toilet system.

Thereby, a user or a healthcare professional may in a particularly simple and easy to understand manner be aided in interpreting the data obtained by the sensor devices and the result obtained by the data analysis device.

A further advantage lies in that visualization of the data may thus be performed automatically and without any need for physical contact with the excretion or the toilet system. Thereby, time and costs otherwise spent by healthcare personal, not only on analysis and visualization, but also on associated sterilization and cleaning, is eliminated and the hygiene and durability of the toilet system is further increased.

In an embodiment, the toilet system further comprises an actuator configured to allow a user to indicate a type of excretion excreted into the bowl by the user of the toilet system and to transmit a signal indicative of the user's indication to an analysis device.

Thereby, further data for determining the type of excretion excreted into a bowl of a toilet system is provided in a particularly simple, straight forward and cost efficient manner.

In an embodiment, the toilet system further comprises an actuator configured to allow a user to activate one or more of the toilet system and the analysis device before use of the toilet system.

Such an actuator may for instance be a press button or an actuator, such as a motion sensor, configured to automatically register a user being in a vicinity of or touching the toilet system and activate one or more of the toilet system and the analysis device in reaction to registering a user touching the toilet system or a sound sensor configured to register a sound produced by a user locking the room or toilet stall.

Thereby it becomes possible to allow the toilet system and/or the analysis device to enter into an active state only when the toilet system is to be used by a user, and otherwise be in a passive or inactive state, thereby saving power and costs. In an embodiment, the toilet system further comprises a weight sensor arranged and configured to capture signals indicative of the user's weight before and after excretion, respectively.

Thereby, further data for determining the quantity of excretion excreted into a bowl of a toilet system is provided in a simple and cost efficient manner.

In an embodiment, the first sensor device is an image capturing device arranged in or at any one of the toilet seat and the bowl above a waterline, and the second sensor device is arranged in the outlet of the toilet system in a position downstream of a water seal arranged between the bowl and the outlet of the toilet and comprises a chamber adapted for collecting liquid forced through the water seal and a weight sensor arranged and adapted for measuring the weight of the chamber and liquid contained in the chamber.

In an embodiment, the first sensor device is arranged such as to point downwards at an angle α of between 70 and 80 degrees, such as an angle α of 75 degrees, with the horizontal H or in an angle β of between 20 and 10 degrees, such as an angle β of 15 degrees, with the vertical V, where an angle of 90 degrees in this context corresponds to the vertical V or the direction of gravity.

In an embodiment, the first sensor device is located outside the central axis A of the seat and is rotated around the direction of gravity or vertical V to point in an angle γ of between 25 and 25 degrees, such as an angle γ of 30 degrees, with the central axis A of the seat.

The arrangement of the first sensor device above the waterline in the bowl as well as the above-described angles of the first sensor device each provide for an improved positioning and alignment of the first sensor device in order to obtain the best possible data for ensuring a reliable type recognition. In unison they serve for an optimized positioning and alignment of the first sensor device in order to obtain the best possible data for ensuring a reliable type recognition.

The above and other objects are in a second aspect of the invention achieved by means of a method for determining a type of excretion excreted into a bowl of a toilet system according to any one of the above claims by a user of the toilet system, the method comprising the steps of:
using at least one first sensor device of the toilet system, capturing a signal indicative of a type of excretion excreted into the bowl by a user of the toilet system,
transmitting the captured signal indicative of a type of excretion excreted into the bowl by a user of the toilet system to an analysis device comprising a data processing unit and a data storage unit,
receiving, by means of the data processing unit, one or more signals transmitted by the at least one first sensor device, and
analysing, by means of the data processing unit, the received one or more signals transmitted by the at least one the first sensor device such as to produce a data output indicative of the type of excretion excreted into the bowl by a user of the toilet system.

In an embodiment, the step of analysing comprises performing a comparison with existing data, e.g. from a database, and/or a statistical analysis. The analysis may for instance be performed in a neural network, a principal component analysis (PCA), a decision tree or a clustering.

In an embodiment, the method comprises the further steps of:
using at least one second sensor device of the toilet system, capturing a signal indicative of a quantity of excretion excreted into the bowl by a user of the toilet system,
transmitting the captured signal indicative of a quantity of excretion excreted into the bowl by a user of the toilet system to the analysis device,
receiving, by means of the data processing unit, one or more signals transmitted by the at least one the second sensor device, and
analysing, by means of the data processing unit, the received one or more signals transmitted by the at least one the second sensor device such as to produce a data output indicative of the quantity of excretion excreted into the bowl by a user of the toilet system.

In an embodiment of the method, the data analysis device further comprises a data visualization unit, and the method further comprises the step of visualizing on the data visualization unit, using the data processing unit, the data output indicative of the type of excretion excreted into the bowl by a user of the toilet system and/or the data output indicative of the quantity of excretion excreted into the bowl by a user of the toilet system.

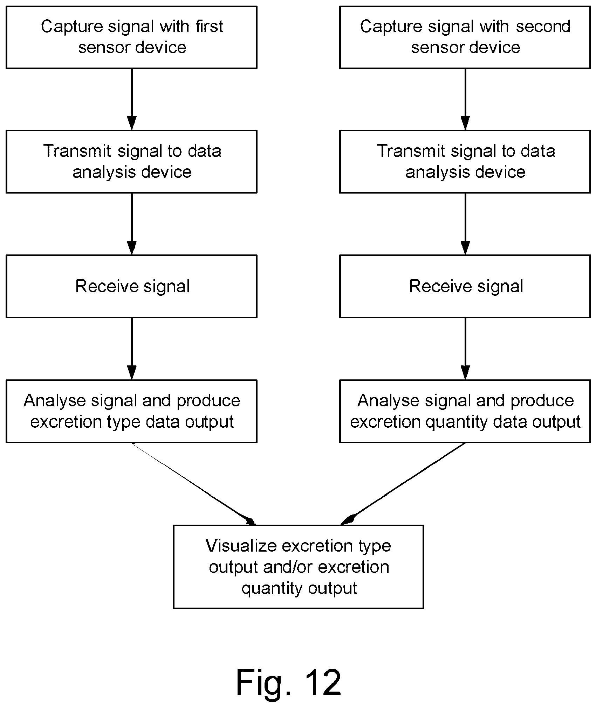

In order to avoid repetition, it is here noted that Fig. 12 schematically illustrates the steps of a method according to the second aspect of the invention. It is further noted that the four steps relating to the second sensor device and shown in the upper right hand side of Fig. 12 are optional steps in line with the embodiments of a method according to the second aspect of the invention as described above. For further details, reference is made to the Example enclosed in the detailed description following below.

Further aspects of the invention include the following.

A toilet system comprising a bowl and an outlet, the toilet system being adapted for discerning between types of excretion excreted into the bowl by a user of the toilet system, the toilet system further comprising:
at least one first sensor device configured to capture at least one signal indicative of a type of excretion excreted into the bowl by a user of the toilet system.

A toilet system comprising a bowl and an outlet, the toilet system being adapted for discerning between types of excretion excreted into the bowl by a user of the toilet system, the toilet system further comprising:
at least one first sensor device configured to capture at least one signal indicative of a type of excretion excreted into the bowl by a user of the toilet system, the at least one first sensor device being configured to measure sounds.

The at least one first sensor device may further be configured to transmit the at least one signal to a data analysis device.

A toilet system comprising a bowl and an outlet, the toilet system being adapted for discerning between types of excretion excreted into the bowl by a user of the toilet system, the toilet system further comprising:
at least one first sensor device configured to capture at least one signal indicative of a type of excretion excreted into the bowl by a user of the toilet system and to transmit the at least one signal to a data analysis device, and
a data analysis device, the data analysis device comprising a data processing unit and a data storage unit, the data processing unit being configured to:
   receive one or more signals transmitted by the at least one first sensor device, and
   analyse the received one or more signals such as to produce a data output indicative of at least the type of excretion excreted into the bowl by a user of the toilet system.

It is noted that the invention relates to all possible combinations of features recited in the claims.

### Brief Description of Drawings

In the following description embodiments of the invention will be described with reference to the schematic drawings, in which
Fig. 1 is a schematic cross-sectional side view of a toilet system according to the invention comprising a first sensor device configured to measure type of excretion and a second sensor device configured to measure quantity of excretion,
Fig. 2 is a schematic illustration of an analysis device for a toilet system according to the invention and shown physically separate from the remaining components of the toilet system shown in Fig. 1,
Fig. 3 is a schematic cross-sectional illustration of a first sensor device according to the invention,
Fig. 4 is a schematic cross-sectional side view of a bowl of a toilet system according to the invention illustrating the difference in sound caused by excretion in dependence of the place in the bowl of the toilet system which the excretion hits.
Fig. 5 is a schematic cross-sectional side view analogous to Fig. 4, where the excretion is urine,
Fig. 6 is an exemplary plot illustrating a signal captured by the first sensor device in a situation according to Fig. 5, the signal being plotted as amplitude A as function of time t,
Fig. 7 is a schematic cross-sectional side view analogous to Fig. 4, where the excretion is faeces,
Fig. 8 is an exemplary plot illustrating a signal captured by the first sensor device in a situation according to Fig. 6, the signal being plotted as amplitude A as function of time t,
Fig. 9 is an exemplary plot illustrating the result of a statistical analysis performed on the signal according to Fig. 6 to provide at any given time the probabilities concerning different types of excretion entering the bowl of the toilet system according to the invention,
Fig. 10 is an exemplary plot illustrating the result of a statistical analysis performed on the signal according to Fig. 8 to provide at any given time the probabilities concerning different types of excretion entering the bowl of a toilet system according to the invention,
Fig. 11 is an exemplary graph illustrating the result of combining the plots of Figs. 8 to 10 with a measurement captured by the second sensor device of a toilet system according to the invention and performing an analysis on the data to assess the type and the quantity of excretion entering the bowl of a toilet system according to the invention, the result of the analysis being plotted as quantity as function of time, and
Fig. 12 illustrates schematically an embodiment of a method according to the invention.
Fig. 13 illustrates schematically a position of the second sensor device configured to measure quantity of excretion on the seat of a toilet system according to the invention, the remaining parts of the toilet system being omitted for simplicity.

### Description of Embodiments

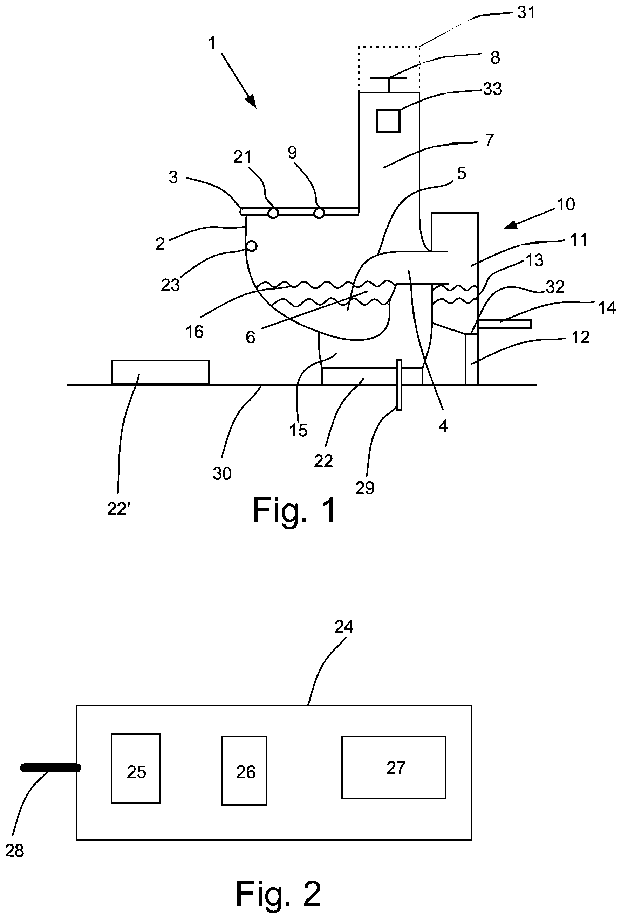

Referring initially to Figs. 1 and 2, a first embodiment of a toilet system 1 according to the invention is shown. The toilet system 1 comprises a bowl 2, an outlet 4 and a seat 3. The outlet 4 further comprises an outlet pipe 5 with a water seal, water trap or U-bend 6. The toilet system 1 may further comprise a toilet tank or flushing cistern 7 with a device 8 adapted for causing the toilet to flush when operated. The device 8 may be an actuator such as a press button or a pull button or a lever. The toilet system 1 may further comprise a basis 15 for connection of the toilet system 1 to a floor 30.

The toilet system 1 comprises a first sensor device 9 and a second sensor device 10. The toilet system 1 may also optionally comprise further sensor devices 11.

The toilet system 1 may be a new separate toilet system 1. Alternatively, the first sensor device 9 and the second sensor device 10 may be mounted on an existing toilet such as to provide a retrofitted toilet system 1. A toilet system 1 according to the invention may thus be provided by retrofitting an existing toilet by providing a first sensor device 9 and a second sensor device 10 and mounting the first sensor device 9 and the second sensor device 10 on the existing toilet.

Generally, the first sensor device 9 is configured to capture a signal indicative of a type of excretion excreted into the bowl 2 by a user of the toilet system 1. The first sensor device is further configured to transmit the captured signal to an analysis device 24, which is described further below with reference to Fig. 2.

The first sensor device 9 is adapted for measuring sound. The first sensor device 9 may be a vibration sensor, such as an acoustic sensor or a microphone. Suitable types of microphones include dynamic microphones, piezoelectric microphones, crystal microphones, fiber-optic microphones and MEMS microphones. The first sensor device 9 may be wired or wireless.

The first sensor device 9 is arranged in or on the seat 3. Alternatively, the first sensor device 9 may be arranged in the bowl 2 or on an inner surface of the bowl 2. In any event the first sensor device 9 is arranged in a position enabling the first sensor device 9 to capture the sounds caused by or in connection with a user excreting an excretion, e.g. voiding his or her bowel and/or bladder, into the bowl 2. The first sensor device 9 is arranged above the upper water level 16 in the bowl, but may alternatively also be arranged below the upper water level 16 in the bowl.

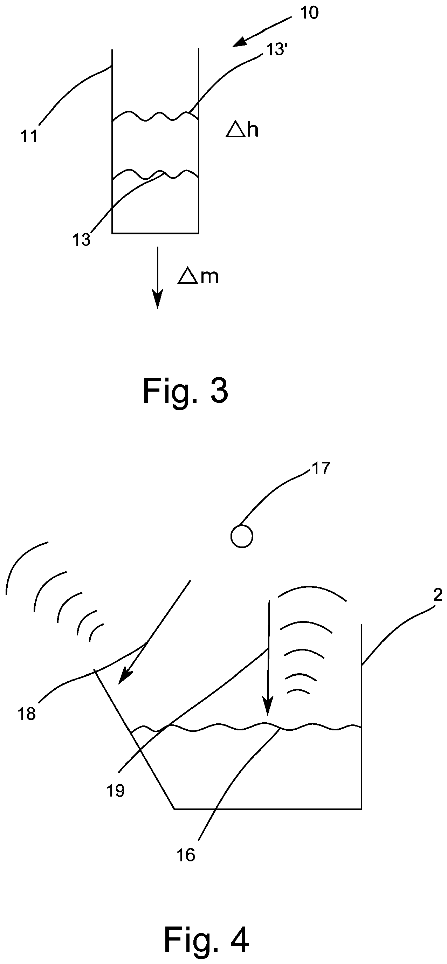

Using a first sensor device 9 adapted for measuring sound and thus capturing a sound signal indicative of a type of excretion excreted into the bowl 2 by a user of the toilet system 1 provides for a high degree of detail in the data captured. Different types of excretion, particularly urine and faeces, respectively, thus generate different sound profiles of the sound signal. It may even be possible through careful analysis to discern between different consistencies of faecal excretions, e.g. solid faeces and diarrhoea, which may be of importance for diagnostic purposes. The sound profile further changes in accordance with the part of the bowl hit by the excretion. As is illustrated in Fig. 4, a sound signal with one sound profile results from the excretion 17 hitting the bowl 2 directly (arrow 18), while a sound signal with another sound profile results from the excretion 17 hitting the water surface 16 in the bowl 2. Furthermore, using a first sensor device 9 adapted for measuring sound and thus capturing a sound signal even makes it possible to identify and remove, during analysis, unwanted data, such false positives resulting from for instance paper or vomit or something else different from excretion of faeces or urine being excreted into the bowl 2 or even the user merely passing gas, or nothing happening at all.

The at least one first sensor device 9 may also comprise or be any one or more of a pressure sensor, a radar, an image capturing device, a distance sensor, a LIDAR, an acoustic distance sensor, and a flow rate sensor, such sensors also being capable of providing a signal indicative of a type of excretion excreted into the bowl 2 by a user of the toilet system 1.

Generally, the second sensor device 10 is configured to measure a volume of liquid indicative of a quantity of excretion excreted into the bowl 2 by a user of the toilet system 1. The second sensor device 10 may further be configured to transmit the captured signal to an analysis device 24 (Fig. 2). The second sensor device 10 may be arranged and configured to measure the amount of liquid forced through the water seal or U-bend 6 when a user voids his or her bowel and/or bladder into the bowl 2. In virtue of Archimedes' law, the amount of liquid forced through the water seal or U-bend 6 when a user voids his or her bowel and/or bladder into the bowl 2 is equivalent, or at least close to equivalent depending on the buoyancy of the excreted material, to the quantity of excretion excreted into the bowl 2 of the user.

The second sensor device 10 is in the embodiment shown in Fig. 1 arranged in the outlet 4 of the toilet in a position downstream of the water seal 6. The second sensor device 10 comprises a chamber 11 for collecting the water 13 forced through the water seal 6 as a result of a user depositing an excretion in the bowl 2 of the toilet system 1. The second sensor device 10 further comprises a measuring unit 14 configured to measure the quantity of liquid 13 collected in the chamber 11. The second sensor device 10 further comprises an outlet 12 arranged in a bottom area of the chamber 11 and connected to a sewer system or the like. The second sensor device 10 may still further comprise a closure mechanism 32, such as a valve, a flap or a shutter, configured to be closed while the measuring unit 14 measures the quantity of liquid 13 collected in the chamber 11 and to be opened subsequently to allow the chamber 11 to be emptied and the toilet system 1 to be flushed when the toilet system 1 is flushed.

Referring also to Fig. 3, the quantity of liquid collected in the chamber 11 may for instance be measured as a difference, ΔH, in depth or water level between a depth 13 before and a depth 13' after an excretion has been excreted into the bowl 2 of the toilet system 1. The quantity of liquid collected in the chamber 11 may also be measured as a difference, ΔM, in mass of the water in the chamber with contained water before and after an excretion has been excreted into the bowl 2 of the toilet system 1, respectively, or as a difference in volume, ΔV, of water exiting the chamber 11 when the closure mechanism 32 is opened after an excretion has been excreted into the bowl 2 of the toilet system 1.

The second sensor device 10 may alternatively be arranged in the bowl 2 or the outlet 4. Such an embodiment is especially advantageous in case of retrofitting an existing toilet with a first sensor device 9 and a second sensor device 10 since it makes the installation of the second sensor device 10 particularly easy and cost efficient. In any event the second sensor device 10 is arranged in a position enabling the second sensor device 10 to measure the amount of liquid forced through the water seal or U-bend 6 when a user excretes an excretion into the bowl 2.

The second sensor device 10 may be or comprise any one or more of a weight sensor, a level sensor and a flow sensor. By level sensor one may understand any type of distance measuring device being mounted in a fixed position, here with respect to the surface of the liquid collected in the chamber 11, whether above or below said surface.

The measuring unit 14 of the second sensor device 10 may comprise a flow sensor, a weight sensor, a depth gauge, a volume sensor or the like.

The toilet system 1 may in some embodiments also comprise one or more further sensor devices, such as sensor device 23 and/or sensor devices 22 and 22' shown on Fig. 1.

The one or more further sensor devices 23 may comprise any one or more of a pressure sensor, a radar, an image capturing device, and a flow rate sensor, such sensors being capable of providing further data or capturing further signals indicative of a type of excretion excreted into the bowl 2 by a user of the toilet system 1.

The one or more further sensor devices 22, 22' are weight sensors each configured to capture a signal indicative of the weight of the user before and after depositing an excretion into the bowl 2 of the toilet system 1. The further sensor device 22 represents a weight sensor arranged under the toilet system 1, particularly under the bowl 2 and/or the basis 15 of the toilet system 1. The further sensor device 22' represents a weight sensor arranged under the feet of a user sitting on the seat 3 of the toilet system 1. The further sensor devices 22 and 22' may both be provided and supplement one another. Alternatively, only one of the further sensor devices 22 and 22' may be provided. It is also feasible to arrange such a weight sensor in or under the seat 3.

The toilet system 1 may further comprise an actuator 33 configured to allow a user to indicate a type of excretion excreted or to be excreted into the bowl 2 by the user of the toilet system 1. The actuator 33 may further be configured to transmit a signal indicative of the user's indication to the data analysis device 24 (Fig. 2). The actuator 33 may for instance be a button, a lever or a touch screen.

The toilet system 1 may further comprise an actuator 21 configured to allow a user to activate the toilet system 1 and/or the data analysis device 24 (Fig. 2) before use of the toilet system 1. The actuator 21 may for instance be a pressure switch or a touch sensor arranged in the seat 3 such as to be automatically actuated when a user sits on the seat 3. Alternatively, the actuator 21 may be a button, a lever, a touch screen, a motion sensor or another suitable type of sensor. In the latter case it is also feasible that the actuators 21 and 33 may be integrated with or arranged next to one another, for instance by means of the same touch screen. Another alternative is an actuator 21 adapted for electronically recognizing, e.g. by means of face recognition, a person or patient for whom measurements of excretion output is desired.

The toilet system 1 may further comprise a connection 29 to a data analysis device 24. Referring to Fig. 2, the data analysis device 24 likewise comprises a connection 28 to the toilet system 1. The connections 28 and 29 may be wired or wireless. Also, the data analysis device 24 may be an external device or it may be integrated with the toilet system, for instance but not limited to on top of the cistern 7 as indicated by the box 31 shown in Fig. 1 with dotted line.

The data analysis device 24 comprises a data processing unit 25, a data storage unit 26 and a data visualization unit 27. The data visualization unit 27 may for instance be a display. The data visualization unit 27 is an optional unit.

The data analysis device 24 may be integrated in or with another component of the toilet system 1, such as the cistern 7 or the bowl 2, or where provided the second sensor device 10. Alternatively, the data analysis device 24 may be physically separated from the remaining parts of the toilet system, an example being a suitable type of computer, e.g. placed on a table or mounted on a wall, connected to the first sensor device 9, and where provided the second sensor device 10 and/or the further sensor devices 22, 22', 23, to enable data transfer to and from the said sensor devices.

The data analysis device 24 is configured to receive one or more signals captured using the first sensor device 9 and, where provided, the second sensor device 10 and/or the further sensor devices 22, 22', 23 and transmitted by the first sensor device 9 and, where provided, the second sensor device 10 and/or the further sensor devices 22, 22', 23. The data analysis device 24 is further configured to analyse, using the data processing unit 25, the received signals such as to produce a data output indicative of the type of excretion and/or the quantity of excretion excreted into the bowl 2 by a user of the toilet system 1.

The data analysis device 24 is further configured to display, using the data processing unit 25 and on the data visualization unit 27, a data output indicative of the type and/or the quantity of excretion excreted into the bowl 2 by a user of the toilet system 1. The data analysis device 24 may further be configured to store, in the data storage device 26, the received signals and/or the produced data output indicative of the type of excretion and/or the quantity of excretion excreted into the bowl 2 by a user of the toilet system 1. The data storage device 26 may further contain data usable for performing a comparison with data retrieved from the received signals in order to further improve the certainty of the determination of the type of excretion excreted into the bowl 2 by a user of the toilet system 1. Such data may e.g. be data from previous analyses and/or data retrieved from other sources, such as other similar toilet systems. The data may be stored in a database provided in the data storage device 26.

Referring again to Figs. 1 and 2, the toilet system 1 in a particular embodiment comprises a first sensor device 9 and a second sensor device 10.

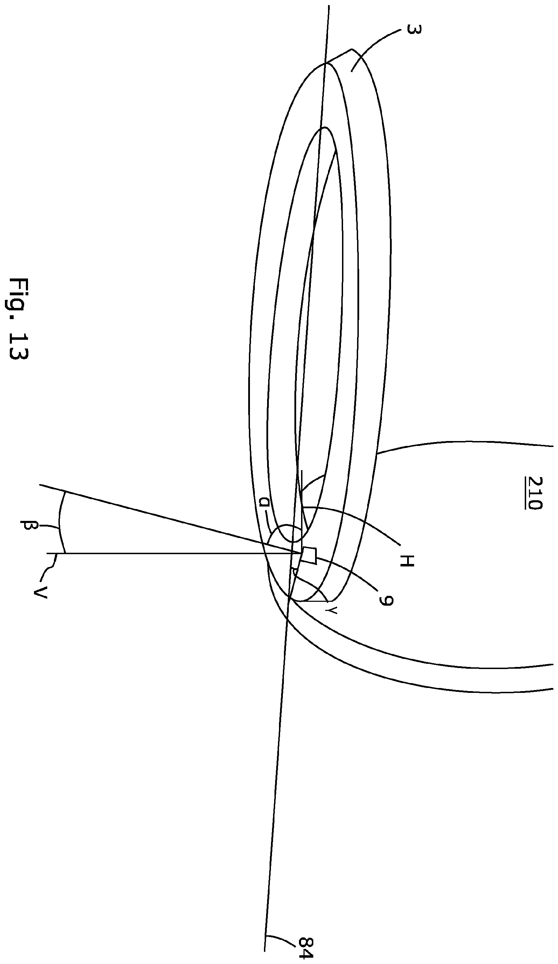

The first sensor device 9 is an image capturing device. The first sensor device 9 is arranged in or on the seat 3 of the toilet system 1. In any event the first sensor device 9 is arranged in a position enabling the first sensor device 9 to capture images showing excretion, e.g. by a user voiding his or her bowel and/or bladder, and/or other things, e.g. toilet paper, being disposed into the bowl 2. The first sensor device 9 is arranged above the upper water level 16 in the bowl. Referring also to Fig. 13, the first sensor device 9 is arranged such as to point downwards at an angle α of between 70 and 80 degrees, such as an angle α of 75 degrees, with the horizontal H, or in other words an angle β of between 20 and 10 degrees, such as an angle β of 15 degrees, with the vertical V. Thus, an angle of 90 degrees in this context corresponds to the vertical V or the direction of gravity. Furthermore, the first sensor device 9 is located outside the central axis A of the seat 3 and is rotated around the direction of gravity or vertical V to point in an angle γ of between 25 and 25 degrees, such as an angle γ of 30 degrees, with the central axis A of the seat 3.

The second sensor device 10 is arranged in the outlet 4 of the toilet system 1 in a position downstream of the water seal 6. The second sensor device 10 comprises a chamber 11 for collecting the water 13 forced through the water seal 6 as a result of a user depositing an excretion in the bowl 2 of the toilet system 1. The second sensor device 10 further comprises an outlet 12 arranged in a bottom area of the chamber 11 and connected to a sewer system or the like. Alternatively, the second sensor device 10 may comprise a so-called outlet pump, i.e. an outlet (not shown on fig. 1) arranged in a top area of the chamber 11 and connected to a sewer system or the like as well as a pumping device arranged and adapted for emptying the chamber 11 by pumping the contents of the chamber 11 into the outlet and thus into the sewer system. The second sensor device 10 furthermore comprises a measuring unit 14 in the form of a weight sensor arranged and adapted to measure the weight of the chamber 11. In this way, the second sensor device 10 takes advantage of the fact that due to the construction of the water seal 6 and due to Archimede's law, the amount of liquid forced through the water seal 6 of the toilet system 1 is equivalent to the amount of excretion disposed into the bowl 2 of the toilet system 1.

The second sensor device 10 may still further comprise a closure mechanism 32, such as a valve, a flap or a shutter, configured to be closed while the measuring unit 14 measures the weight of the chamber 11 and the liquid 13 collected in the chamber 11 and to be opened subsequently to allow the chamber 11 to be emptied and the toilet system 1 to be flushed when the toilet system 1 is flushed.

The data analysis device 24 (cf. Fig. 2) is in this particular embodiment configured to receive one or more signals captured using the first sensor device 9 and the second sensor device 10 and transmitted by the first sensor device 9 and the second sensor device 10. The data analysis device 24 is further configured to analyse, using the data processing unit 25, the received signals such as to produce a data output indicative of the type of excretion and/or the quantity of excretion excreted into the bowl 2 by a user of the toilet system 1. To this end, the data analysis unit 25 and the data processing unit 25 uses suitable image analysis and/or image recognition software.

The analysis to determine a type of excretion or deposition into the bowl 2 of the toilet system 1 may be completed using a statistical method, such as a neural network, provided in or to the data analysis device 24. The first step for doing the training, or calibration, of the neural network, is to acquire relevant training data. To this end, a sampling of several thousand toilet visits using the above-described sensors has been conducted. The second step is to extract exemplary data for type determination. In this case, the exemplary data is data from the first sensor device 9 which may depict one or more of, e.g., an empty bowl, urine, feces, and paper. The extraction is performed manually by a trained person. The training ensures is that there is provided clear definitions of each category, e.g., empty bowl 2 containing only water, or bowl 2 with urine, feces, and/or paper. The definitions, their interpretation, and the final type recognition are closely linked. The extraction process comprises naming computer directories, naming the relevant categories, and subsequently moving relevant imaging data to the corresponding directory. The statistical model can then be calibrated to classify data from the first sensor device 9 to belong to one of the different categories, for instance by means of the previously described computer directories. Once the statistical model is calibrated, it can organize imaging data as it arrives from the first sensor device 9. Therefore, a time stamped type determination can also be obtained.

The first sensor device 9 and the data analysis device 24 can now be used to tell if there is urine or feces in the bowl 2. The total quantity excreted, as measured by the second sensor device 10, can then be mapped to a category, particularly either urine, feces, or a combination. Changes in total quantity can also be mapped to each category, thus giving the quantity of urine and feces separately. However, in cases where a more considerable amount of feces arrives before urine, it may be difficult to recognize the urine precisely. To correct this error, the same processes can be repeated for data indicative of the active excretory action, e.g., urination and defecation. In principle, both imaging and audio can be input for the excretory action determination.

### Experiment and example

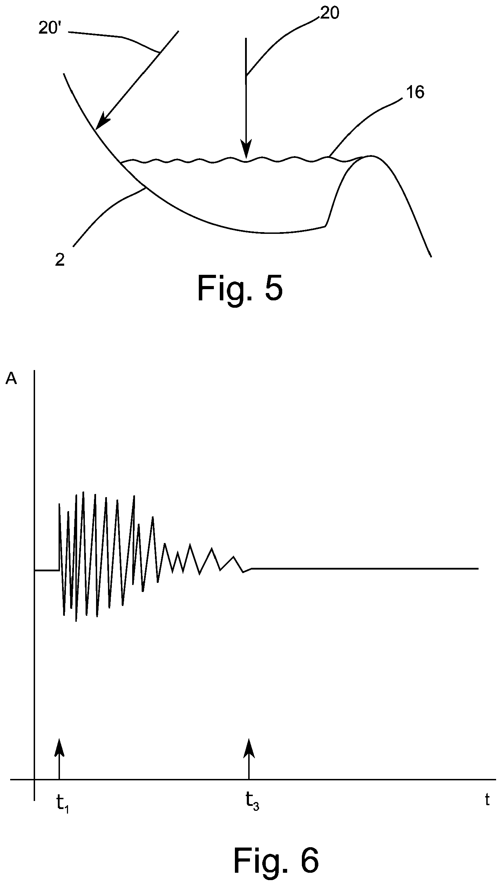

Turning now to Figs. 5 to 11 a general example of data capture and analysis using a toilet system 1 according to the present invention and as described above will be given. In order to enable identification of the type of excretion as described below, sound capture measurements of more than one thousand different excretions were made to provide background data for comparison purposes and statistical analysis. The background data may be stored in a database, which may be provided in the storage device 26 of the analysis device 24 (Fig. 2). Further data may be added to the database as new measurements are made. The experiment has shown that it is in fact possible to determine the type of excretion based on sound measurements of the excretion with a sufficiently high certainty to be usable in practice. An analogous or similar approach may be made to provide background quantity information for comparison purposes and statistical analysis.

Fig. 5 illustrates a bowl 2 of a toilet system 1 according to the invention into which a user excretes urine 20, 20', and thus a thin, liquid excretion, directly onto the surface of the bowl 2 and into the water 16 in the bowl, respectively.

Fig. 6 illustrates a transform of the captured sound signal, or more precisely a plot of the signal amplitude A as a function of elapsed time t of a sound signal captured by means of the first sensor device 9 of the toilet system 1 and corresponding to the excretion illustrated in Fig. 5. As may be seen the sound profile extends over a relatively long period of time Δt = t3 - t1. At t = t1, the amplitude of the sound signal is large, possibly peaking, and as time elapses towards t = t3, the amplitude gradually decreases towards zero. Such an acoustic image is characteristic for the excretion of urine.

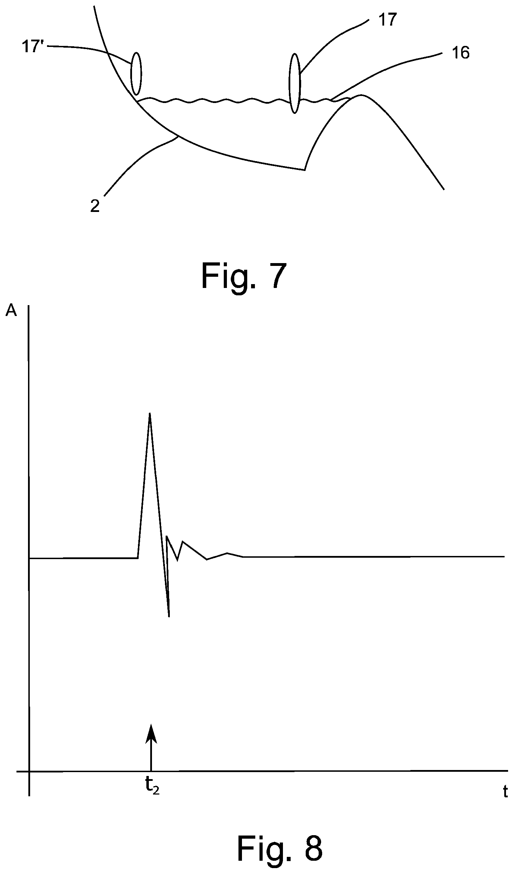

Fig. 7 illustrates a bowl 2 of a toilet system 1 according to the invention into which a user excretes faeces 17, 17', and thus a relatively solid excretion, directly onto the surface of the bowl 2 and into the water 16 in the bowl, respectively.

Fig. 8 illustrates a transform of the captured sound signal, or more precisely a plot of the signal amplitude A as a function of elapsed time t of a sound signal captured by means of the first sensor device 9 of the toilet system 1 and corresponding to the excretion illustrated in Fig. 7. As may be seen the sound profile extends over a short period of time around t = t2, being the time of impact of the faeces with the water 16 of the surface of the bowl 2, as the case may be. As may be seen, the amplitude of the sound signal increases and decreases rapidly on each side of and close to t = t2. Such an acoustic image is characteristic for the excretion of faeces.

Thus, objects with different characteristics and/or hitting surfaces with different characteristics, and/or being dropped into a container - such as the bowl 2 - in different ways, will produce different vibrations and therefore different sounds and acoustic images.

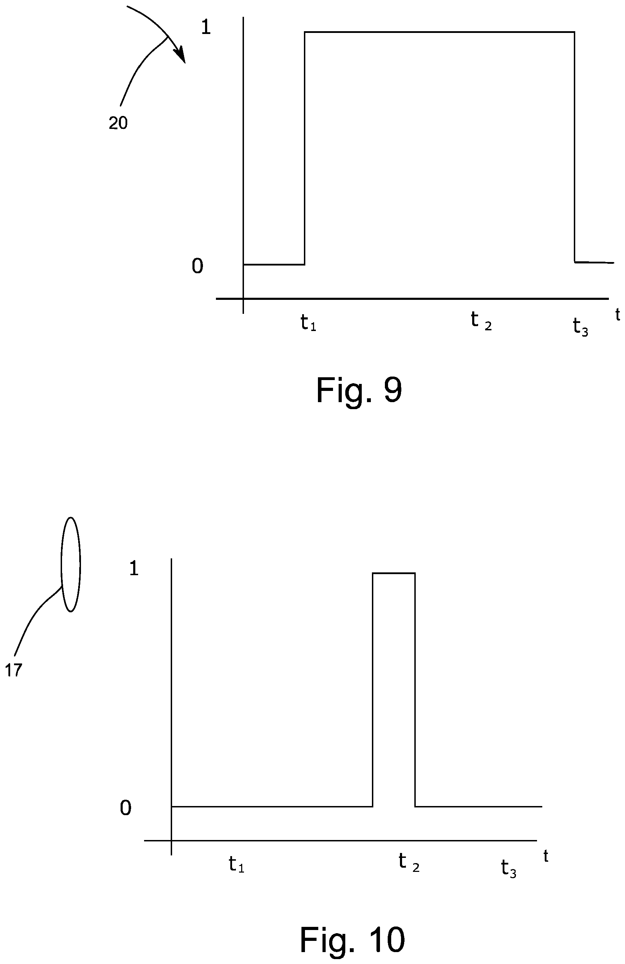

The transforms or plots shown in Figs. 6 and 8, respectively, may now be analysed, such as analysed statistically, for instance in a neural network, a principal component analysis (PCA), a decision tree or a clustering. Thereby, the probabilities concerning different objects, solids or liquids excreted into the bowl 2 of the toilet system 1 at any given time may be provided. The result of such an analysis of the transforms or plots shown in Figs. 6 and 8, respectively, are shown in Figs. 9 and 10, respectively.

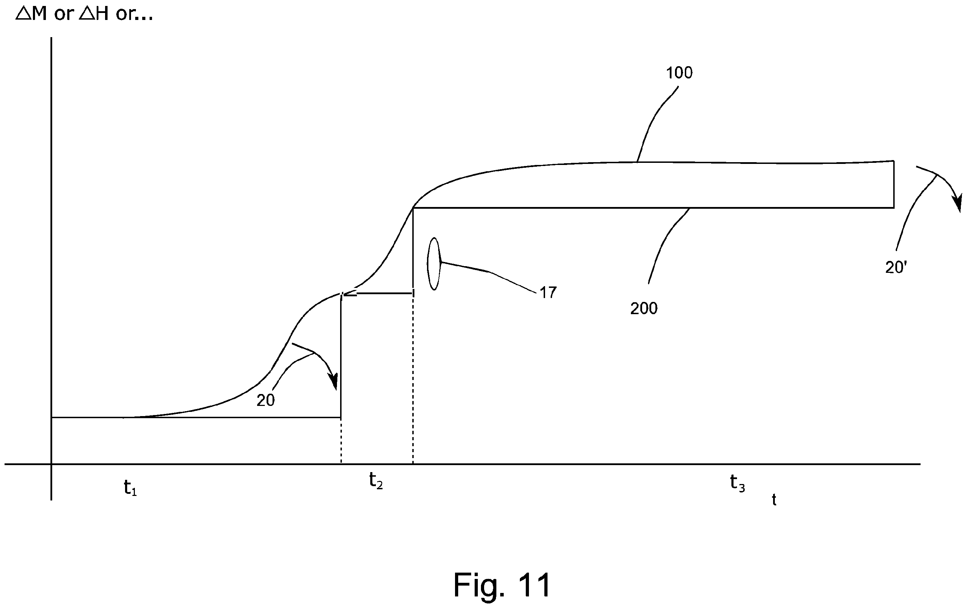

Finally, as illustrated in Fig. 11, the probabilities shown in Figs. 9 and 10 may, combined with the measures of mass or liquid level of the container or any connected container obtained using the second sensor device 10, be used to assess how much of an object, solid or liquid has entered the bowl 2 of the toilet system 1 as well as when it entered the bowl 2 of the toilet system 1. Fig. 11 illustrates an example of a visualization of the result of such an analysis. Curve 100 is a plot of the change in water level (ΔH), in mass (ΔM), in volume (ΔV) or in flow rate according to measurement principle used as a function of time elapsed as indicated by the signal captured by the second sensor device 10. Curve 200 indicates the accumulated mass or quantity of the excretions. The inserted symbols 20, 17 and 20' indicates the result of the statistical analysis made on the data provided by the first sensor device 9 and providing probabilities as described above in relation to Figs. 6 and 8, and thus the identified respective types of excretion.

As shown in Fig. 11, the exemplary analysis reveals that with a high probability in the time interval between t = t1 and t = t2, a first amount of urine 20 was excreted into the bowl 2, in the time interval just around t = t2, an amount of faeces 17 was excreted into the bowl 2, and in the time interval between t = t2 and t = t3, a second amount of urine 20', being smaller than the first amount of urine 20, was excreted into the bowl 2.

The combination of a sound signal provided by the first sensor device 9 and a further signal provided by the second sensor device 10 will increase the True Negative Rate of the analysis. For instance, sounds such as those of flatulence is not always associated with a change in mass or volume of the container and may thus be removed upon identification.

Compared to only using the mass or volume, the experiments have shown that a better certainty can be obtained as to what liquid or solid is entering the container. For instance, it is possible to differ between liquids poured in at the same rate, whether the liquids hit different parts, but also if the width of the liquid stream is different but the flow the same. The same applies to solids.

Furthermore, the experiments have shown that not only may different types of excretion, particularly urine and faeces, respectively, be identified. It is even possible to discern between different consistencies of faecal excretions, e.g. solid faeces and diarrhoea, which may be of importance for diagnostic purposes. It has further been shown to be possible to identify and remove, during analysis, false positives resulting from for instance paper or vomit or something else different from excretion of faeces or urine being excreted into the bowl 2 or even the user merely passing gas, or nothing happening at all.

The person skilled in the art realizes that the present invention by no means is limited to the preferred embodiments described above. On the contrary, many modifications and variations are possible within the scope of the appended claims.

### List of reference numerals

- 1: Toilet
- 2: Bowl
- 3: Seat
- 4: Outlet
- 5: Outlet pipe
- 6: Water seal
- 7: Cistern
- 8: Actuator (flushing device)
- 9: First sensor device (type)
- 10: Second sensor device (quantity)
- 11: Compartment
- 12: Outlet of compartment
- 13: Water held back in compartment
- 14: Actuator
- 15: Base
- 16: Water in bowl
- 17: Object (faeces)
- 18: Arrow
- 19: Arrow
- 20: Object (urine)
- 21: Sensor (for actuating system)
- 22: Weight sensor
- 23: Further sensor
- 24: Data analysis device
- 25: Data processing device
- 26: Data storage device
- 27: Data visualization unit
- 28: Connection to toilet
- 29: Connection to data analysis device
- 30: Floor
- 31: Box
- 32: Closure mechanism
- 33: Actuator

## Claims

1. A toilet system (1) comprising a bowl (2) and an outlet (4), the toilet system (1) being adapted for discerning between types of excretion excreted into the bowl (2) by a user of the toilet system (1), the toilet system (1) further comprising:
- at least one first sensor device (9) configured to capture at least one signal indicative of a type of excretion excreted into the bowl (2) by a user of the toilet system (1), and
- at least one second sensor device (10) configured to capture at least one signal indicative of a quantity of excretion excreted into the bowl (2) by a user of the toilet system (1), and **characterized in that**
the second sensor device (10) is arranged in the outlet (4) of the toilet system (1) in a position downstream of a water seal (6) arranged between the bowl (2) and the outlet (4) of the toilet system (1) and comprises a chamber (11) adapted for collecting liquid (13) forced through the water seal (6) and a measuring unit (14) arranged and adapted for measuring the quantity of liquid (13) collected in the chamber (11).

2. A toilet system according to claim 1, wherein the at least one first sensor device (9) is configured to measure sounds, and/or
wherein the at least one first sensor device (9) comprises an acoustic sensor or a microphone or a vibration sensor.

3. A toilet system according to any one of the above claims, wherein the at least one first sensor device (9) is arranged in or at any one of the toilet seat (3), the bowl (2) above a waterline (16) and the bowl (2) below the waterline (16).

4. A toilet system according to any one of the above claims, wherein the at least one first sensor device (9) further comprises any one or more of a pressure sensor, a radar, an image capturing device, a capacitive sensor, and flow rate sensor, and/or
wherein the at least one first sensor device (9) is further configured to transmit the at least one signal indicative of a type of excretion excreted into the bowl (2) by a user of the toilet system (1) to a data analysis device (24).

5. A toilet system according to any one of the above claims, wherein the at least one second sensor device (10) is configured to measure a volume of liquid indicative of a quantity of excretion excreted into the bowl (2) by a user of the toilet system (1), and/or
wherein the at least one second sensor device (10) is arranged in the outlet (4) of the toilet system (1) in a position downstream of a water seal (6) arranged between the bowl (2) and the outlet (4) of the toilet, and/or
wherein the at least one second sensor device (10) is further configured to transmit the at least one signal indicative of a quantity of excretion excreted into the bowl (2) by a user of the toilet system (1) to a data analysis device (24).

6. A toilet system according to any one of the above claims, and further comprising a data analysis device (24), the data analysis device (24) comprising a data processing unit (25) and a data storage unit (26), the data processing unit (25) being configured to:
- receive one or more signals transmitted by the at least one first sensor device (9), and
- analyse the received one or more signals such as to produce a data output indicative of at least the type of excretion excreted into the bowl (2) by a user of the toilet system (1).

7. A toilet system according to claim 6, wherein the data analysis device (24) is further configured to:
- receive one or more signals transmitted by the at least one second sensor device (10), and
- analyse, using the data processing unit (25), the received one or more signals such as to produce a data output indicative of the quantity of excretion and the quantity of excretion excreted into the bowl (2) by a user of the toilet system (1),
and/or
wherein the data analysis device (24) further comprises a data visualization unit (27), and the data processing unit (25) is further configured to visualize on the data visualization unit (27) the data output indicative of one or more of the type of excretion excreted into the bowl (2) by a user of the toilet system (1) and the quantity of excretion excreted into the bowl (2) by a user of the toilet system (1).

8. A toilet system according to any one of the above claims, and further comprising any one or more of:
- an actuator (33) configured to allow a user to indicate a type of excretion excreted into the bowl (2) by the user of the toilet system (1) and to transmit a signal indicative of the user's indication to an analysis device (24),
- an actuator (21) configured to allow a user to activate the toilet system (1) and/or the analysis device (24) before use of the toilet system (1), and
- a weight sensor (22, 22') arranged and configured to capture signals indicative of the user's weight before and after excretion, respectively.

9. A toilet system according to any one of the above claims, wherein the first sensor device (9) is an image capturing device arranged in or at any one of the toilet seat (3) and the bowl (2) above a waterline (16).

10. A toilet system according to claim 9, wherein the first sensor device (9) is arranged such as to point downwards at an angle (α) of between 70 and 80 degrees, such as an angle (α) of 75 degrees, with the horizontal (H) or in an angle (β) of between 20 and 10 degrees, such as an angle (β) of 15 degrees, with the vertical (V), where an angle of 90 degrees in this context corresponds to the vertical (V) or the direction of gravity.

11. A method for determining a type of excretion excreted into a bowl (2) of a toilet system (1) according to any one of the above claims by a user of the toilet system (1), the method comprising the steps of:
- using at least one first sensor device (9) of the toilet system (1), capturing a signal indicative of a type of excretion excreted into the bowl (2) by a user of the toilet system (1),
- transmitting the captured signal indicative of a type of excretion excreted into the bowl (2) by a user of the toilet system (1) to an analysis device (24) comprising a data processing unit (25) and a data storage unit (26),
- receiving, by means of the data processing unit (25), one or more signals transmitted by the at least one first sensor device (9), and
- analysing, by means of the data processing unit (25), the received one or more signals transmitted by the at least one first sensor device (9) such as to produce a data output indicative of the type of excretion excreted into the bowl (2) by a user of the toilet system (1).

12. A method according to claim 11, and comprising the further steps of:
- using at least one second sensor device (10) of the toilet system, capturing a signal indicative of a quantity of excretion excreted into the bowl (2) by a user of the toilet system (1),
- transmitting the captured signal indicative of a quantity of excretion excreted into the bowl (2) by a user of the toilet system (1) to the analysis device (24),
- receiving, by means of the data processing unit (25), one or more signals transmitted by the at least one second sensor device (10), and
- analysing, by means of the data processing unit (25), the received one or more signals transmitted by the at least one second sensor device (10) such as to produce a data output indicative of the quantity of excretion excreted into the bowl (2) by a user of the toilet system (1),
and/or
wherein the data analysis device (24) further comprises a data visualization unit (27), and the method further comprises the step of:
visualizing on the data visualization unit (27), using the data processing unit (25), the data output indicative of the type of excretion excreted into the bowl (2) by a user of the toilet system (1) and/or the data output indicative of the quantity of excretion excreted into the bowl (2) by a user of the toilet system (1).

## Patentansprüche

1. Toilettensystem (1), das eine Schüssel (2) und einen Auslass (4) umfasst, wobei das Toilettensystem (1) zum Unterscheiden zwischen Typen einer von einem Benutzer oder einer Benutzerin des Toilettensystems (1) in die Schüssel (2) ausgeschiedenen Ausscheidung ausgelegt ist, wobei das Toilettensystem (1) weiter Folgendes umfasst:
- mindestens eine erste Sensorvorrichtung (9), die konfiguriert ist, um mindestens ein Signal, das einen Typ einer von einem Benutzer oder einer Benutzerin des Toilettensystems (1) in die Schüssel (2) ausgeschiedenen Ausscheidung angibt, zu erfassen, und
- mindestens eine zweite Sensorvorrichtung (10), die konfiguriert ist, um mindestens ein Signal, das eine Menge einer von einem Benutzer oder einer Benutzerin des Toilettensystems (1) in die Schüssel (2) ausgeschiedenen Ausscheidung angibt, zu erfassen, und **dadurch gekennzeichnet, dass**
die zweite Sensorvorrichtung (10) in dem Auslass (4) des Toilettensystems (1) an einer Stelle hinter einem zwischen der Schüssel (2) und dem Auslass (4) des Toilettensystems (1) eingerichteten Geruchverschluss (6) eingerichtet ist und eine Kammer (11), die zum Sammeln von durch den Geruchverschluss (6) gedrückter Flüssigkeit (13) ausgelegt ist, und eine Messeinheit (14), die zum Messen der Menge von in der Kammer (11) gesammelter Flüssigkeit (13) eingerichtet und ausgelegt ist, umfasst.

2. Toilettensystem nach Anspruch 1, wobei die mindestens eine erste Sensorvorrichtung (9) konfiguriert ist, um Schall zu messen, und/oder
wobei die mindestens eine erste Sensorvorrichtung (9) einen akustischen Sensor oder ein Mikrofon oder einen Schwingungssensor umfasst.

3. Toilettensystem nach einem der obigen Ansprüche, wobei die mindestens eine erste Sensorvorrichtung (9) in oder an einem von dem Toilettensitz (3), der Schüssel (2) über einer Wasserlinie (16) und der Schüssel (2) unter der Wasserlinie (16) eingerichtet ist.

4. Toilettensystem nach einem der obigen Ansprüche, wobei die mindestens eine erste Sensorvorrichtung (9) weiter einen oder mehrere von einem Drucksensor, einem Radar, einer Bilderfassungsvorrichtung, einem kapazitiven Sensor und einem Durchflusssensor umfasst und/oder
wobei die mindestens eine erste Sensorvorrichtung (9) weiter konfiguriert ist, um das mindestens eine Signal, das einen Typ einer von einem Benutzer oder einer Benutzerin des Toilettensystems (1) in die Schüssel (2) ausgeschiedenen Ausscheidung angibt, an eine Datenanalysevorrichtung (24) zu übertragen.

5. Toilettensystem nach einem der obigen Ansprüche, wobei die mindestens eine zweite Sensorvorrichtung (10) konfiguriert ist, um ein Flüssigkeitsvolumen, das eine Menge einer von einem Benutzer oder einer Benutzerin des Toilettensystems (1) in die Schüssel (2) ausgeschiedenen Ausscheidung angibt, zu messen, und/oder
wobei die mindestens eine zweite Sensorvorrichtung (10) weiter konfiguriert ist, um das mindestens eine Signal, das eine Menge einer von einem Benutzer oder einer Benutzerin des Toilettensystems (1) in die Schüssel (2) ausgeschiedenen Ausscheidung angibt, an eine Datenanalysevorrichtung (24) zu übertragen.

6. Toilettensystem nach einem der obigen Ansprüche, das weiter eine Datenanalysevorrichtung (24) umfasst, wobei die Datenanalysevorrichtung (24) eine Datenverarbeitungseinheit (25) und eine Datenspeichereinheit (26) umfasst, wobei die Datenverarbeitungseinheit (25) für Folgendes konfiguriert ist:
- Empfangen eines oder mehrerer Signale, das/die durch die mindestens eine erste Sensorvorrichtung (9) übertragen worden ist/sind, und
- Analysieren des einen oder der mehreren empfangenen Signale, um eine Datenausgabe, die mindestens den Typ einer von einem Benutzer oder einer Benutzerin des Toilettensystems (1) in die Schüssel (2) ausgeschiedenen Ausscheidung angibt, zu erzeugen.

7. Toilettensystem nach Anspruch 6, wobei die Datenanalysevorrichtung (24) weiter für Folgendes konfiguriert ist:
- Empfangen eines oder mehrerer Signale, das/die durch die mindestens eine zweite Sensorvorrichtung (10) übertragen worden ist/sind, und
- Analysieren des einen oder der mehreren empfangenen Signale unter Verwendung der Datenverarbeitungseinheit (25), um eine Datenausgabe, die die Menge einer Ausscheidung und die Menge einer von einem Benutzer oder einer Benutzerin des Toilettensystems (1) in die Schüssel (2) ausgeschiedenen Ausscheidung angibt, zu erzeugen,
und/oder
wobei die Datenanalysevorrichtung (24) weiter eine Datenvisualisierungseinheit (27) umfasst und die Datenverarbeitungseinheit (25) weiter konfiguriert ist, um die Datenausgabe, die eines oder mehrere von dem Typ einer von einem Benutzer oder einer Benutzerin des Toilettensystems (1) in die Schüssel (2) ausgeschiedenen Ausscheidung und der Menge einer von einem Benutzer oder einer Benutzerin des Toilettensystems (1) in die Schüssel (2) ausgeschiedenen Ausscheidung angibt, auf der Datenvisualisierungseinheit (27) zu visualisieren.

8. Toilettensystem nach einem der obigen Ansprüche, das weiter eines oder mehrere von Folgendem umfasst:
- einem Aktor (33), der konfiguriert ist, um einem Benutzer oder einer Benutzerin das Angeben eines Typs einer von dem Benutzer oder der Benutzerin des Toilettensystems (1) in die Schüssel (2) ausgeschiedenen Ausscheidung zu ermöglichen und ein Signal, das die Angabe des Benutzers oder der Benutzerin angibt, an eine Analysevorrichtung (24) zu übertragen,
- einem Aktor (21), der konfiguriert ist, um einem Benutzer oder einer Benutzerin das Einschalten des Toilettensystems (1) und/oder der Analysevorrichtung (24) vor der Nutzung des Toilettensystems (1) zu ermöglichen, und
- einem Gewichtssensor (22, 22'), der eingerichtet und konfiguriert ist, um Signale, die das Gewicht des Benutzers oder der Benutzerin angeben, vor bzw. nach einer Ausscheidung zu erfassen.

9. Toilettensystem nach einem der obigen Ansprüche, wobei die erste Sensorvorrichtung (9) eine in oder an einem von dem Toilettensitz (3) und der Schüssel (2) über einer Wasserlinie (16) eingerichtete Bilderfassungsvorrichtung ist.

10. Toilettensystem nach Anspruch 9, wobei die erste Sensorvorrichtung (9) so eingerichtet ist, dass sie in einem Winkel (α) von zwischen 70 und 80 Grad, etwa einem Winkel (α) von 75 Grad, mit der Horizontalen (H) oder in einem Winkel (β) von zwischen 20 und 10 Grad, etwa einem Winkel (β) von 15 Grad, mit der Vertikalen (V) nach unten ausgerichtet ist, wobei ein Winkel von 90 Grad in diesem Zusammenhang der Vertikalen (V) oder der Schwerkraftrichtung entspricht.

11. Verfahren zum Bestimmen eines Typs einer in eine Schüssel (2) eines Toilettensystems (1) ausgeschiedenen Ausscheidung nach einem der obigen Ansprüche durch einen Benutzer oder eine Benutzerin des Toilettensystems (1), wobei das Verfahren die folgenden Schritte umfasst:
- Nutzen mindestens einer ersten Sensorvorrichtung (9) des Toilettensystems (1), die ein Signal, das einen Typ einer von einem Benutzer oder einer Benutzerin des Toilettensystems (1) in die Schüssel (2) ausgeschiedenen Ausscheidung angibt, erfasst,
- Übertragen des erfassten Signals, das einen Typ einer von einem Benutzer oder einer Benutzerin des Toilettensystems (1) in die Schüssel (2) ausgeschiedenen Ausscheidung angibt, an eine Analysevorrichtung (24), die eine Datenverarbeitungseinheit (25) und eine Datenspeichereinheit (26) umfasst,
- Empfangen eines oder mehrerer Signale, das/die durch die mindestens eine erste Sensorvorrichtung (9) übertragen worden ist/sind, mittels der Datenverarbeitungseinheit (25) und
- Analysieren des einen oder der mehreren empfangenen Signale, das/die durch die mindestens eine erste Sensorvorrichtung (9) übertragen worden ist/sind, mittels der Datenverarbeitungseinheit (25), um eine Datenausgabe, die den Typ einer von einem Benutzer oder einer Benutzerin des Toilettensystems (1) in die Schüssel (2) ausgeschiedenen Ausscheidung angibt, zu erzeugen.

12. Verfahren nach Anspruch 11, das die folgenden weiteren Schritte umfasst:
- Nutzen mindestens einer zweiten Sensorvorrichtung (10) des Toilettensystems, die ein Signal, das eine Menge einer von einem Benutzer oder einer Benutzerin des Toilettensystems (1) in die Schüssel (2) ausgeschiedenen Ausscheidung angibt, erfasst,
- Übertragen des erfassten Signals, das eine Menge einer von einem Benutzer oder einer Benutzerin des Toilettensystems (1) in die Schüssel (2) ausgeschiedenen Ausscheidung angibt, an die Analysevorrichtung (24),
- Empfangen eines oder mehrerer Signale, das/die durch die mindestens eine zweite Sensorvorrichtung (10) übertragen worden ist/sind, mittels der Datenverarbeitungseinheit (25)
und
- Analysieren des einen oder der mehreren empfangenen Signale, das/die durch die mindestens eine zweite Sensorvorrichtung (10) übertragen worden ist/sind, mittels der Datenverarbeitungseinheit (25), um eine Datenausgabe, die die Menge einer von einem Benutzer oder einer Benutzerin des Toilettensystems (1) in die Schüssel (2) ausgeschiedenen Ausscheidung angibt, zu erzeugen,
und/oder
wobei die Datenanalysevorrichtung (24) weiter eine Datenvisualisierungseinheit (27) umfasst und das Verfahren weiter den folgenden Schritt umfasst:
Visualisieren der Datenausgabe, die den Typ einer von einem Benutzer oder einer Benutzerin des Toilettensystems (1) in die Schüssel (2) ausgeschiedenen Ausscheidung angibt, und/oder der Datenausgabe, die die Menge einer von einem Benutzer oder einer Benutzerin des Toilettensystems (1) in die Schüssel (2) ausgeschiedenen Ausscheidung angibt, auf der Datenvisualisierungseinheit (27) unter Verwendung der Datenverarbeitungseinheit (25) .

## Revendications

1. Système de toilettes (1) comprenant une cuvette (2) et un passage d'évacuation (4), ce système de toilettes (1) étant adapté pour faire la distinction entre des types d'excrétion excrétés dans la cuvette (2) par un utilisateur de ce système de toilettes (1), ce système de toilettes (1) comprenant en outre :
- au moins un premier dispositif de détection (9) configuré de façon à capturer au moins un signal indicatif d'un type d'excrétion excrété dans la cuvette (2) par un utilisateur du système de toilettes (1), et
- au moins un deuxième dispositif de détection (10) configuré de façon à capturer au moins un signal indicatif d'une quantité d'excrétion excrétée dans la cuvette (2) par un utilisateur du système de toilettes (1), et **caractérisé en ce que**
le deuxième dispositif de détection (10) est disposé dans le passage d'évacuation (4) du système de toilettes (1) dans une position en aval d'un siphon hydraulique (6) disposé entre la cuvette (2) et le passage d'évacuation (4) du système de toilettes (1) et comprend une chambre (11) adaptée pour recueillir le liquide (13) propulsé à travers le siphon hydraulique (6) et une unité de mesure (14) disposée et adaptée pour mesurer la quantité de liquide (13) recueillie dans la chambre (11).

2. Système de toilettes selon la revendication 1, dans lequel l'au moins un premier dispositif de détection (9) est configuré de façon à mesurer des sons, et/ou
dans lequel l'au moins un premier dispositif de détection (9) consiste en un capteur acoustique ou un microphone ou un capteur de vibrations.

3. Système de toilettes selon l'une quelconque des revendications précédentes, dans lequel l'au moins un premier dispositif de détection (9) est disposé dans ou au niveau de soit le siège des toilettes (3), soit la cuvette (2) au-dessus d'une ligne d'eau (16) et soit la cuvette (2) en dessous de la ligne d'eau (16).

4. Système de toilettes selon l'une quelconque des revendications précédentes, dans lequel l'au moins un premier dispositif de détection (9) comprend en outre un quelconque ou plusieurs des éléments suivants : un capteur de pression, un radar, un dispositif de capture d'images, un capteur capacitif et un capteur de débit, et/ou
dans lequel l'au moins un premier dispositif de détection (9) est configuré en outre de façon à transmettre l'au moins un signal indicatif d'un type d'excrétion excrété dans la cuvette (2) par un utilisateur du système de toilettes (1) à un dispositif d'analyse de données (24).

5. Système de toilettes selon l'une quelconque des revendications précédentes, dans lequel l'au moins un deuxième dispositif de détection (10) est configuré de façon à mesurer un volume de liquide indicatif d'une quantité d'excrétion excrétée dans la cuvette (2) par un utilisateur du système de toilettes (1), et/ou
dans lequel l'au moins un deuxième dispositif de détection (10) est configuré en outre de façon à transmettre l'au moins un signal indicatif d'une quantité d'excrétion excrétée dans la cuvette (2) par un utilisateur du système de toilettes (1) à un dispositif d'analyse de données (24).

6. Système de toilettes selon l'une quelconque des revendications précédentes, et comprenant en outre un dispositif d'analyse de données (24), ce dispositif d'analyse de données (24) comprenant une unité de traitement de données (25) et une unité de stockage de données (26), l'unité de traitement de données (25) étant configurée de façon à :
- recevoir un ou plusieurs signaux transmis par l'au moins un premier dispositif de détection (9), et à
- analyser le ou les signaux reçus afin de produire une sortie de données indicative du type d'excrétion excrété dans la cuvette (2) par un utilisateur du système de toilettes (1).

7. Système de toilettes selon la revendication 6, dans lequel le dispositif d'analyse de données (24) est configuré en outre de façon à :
- recevoir un ou plusieurs signaux transmis par l'au moins un deuxième dispositif de détection (10), et à
- analyser, en utilisant l'unité de traitement de données (25), le ou les signaux reçus afin de produire une sortie de données indicative de la quantité d'excrétion et la quantité d'excrétion excrétée dans la cuvette (2) par un utilisateur du système de toilettes (1).
et/ou
dans lequel le dispositif d'analyse de données (24) comprend en outre une unité de visualisation de données (27), et l'unité de traitement de données (25) est configurée en outre de façon à visualiser, sur l'unité de visualisation de données (27), la sortie de données indicative du ou des types d'excrétion excrétés dans la cuvette (2) par un utilisateur du système de toilettes (1) et de la quantité d'excrétion excrétée dans la cuvette (2) par un utilisateur du système de toilettes (1) .

8. Système de toilettes selon l'une quelconque des revendications précédentes, et comprenant en outre un quelconque ou plusieurs des éléments suivants :
- un actionneur (33) configuré de façon à permettre à un utilisateur d'indiquer un type d'excrétion excrété dans la cuvette (2) par l'utilisateur du système de toilettes (1) et de façon à transmettre un signal indicatif de l'indication de l'utilisateur à un dispositif d'analyse (24),
- un actionneur (21) configuré de façon à permettre à un utilisateur d'activer le système de toilettes (1) et/ou le dispositif d'analyse (24) avant d'utiliser le système de toilettes (1), et
- un capteur de poids (22, 22') disposé et configuré de façon à capturer des signaux indicatifs du poids de l'utilisateur avant et après l'excrétion, respectivement.

9. Système de toilettes selon l'une quelconque des revendications précédentes, dans lequel le premier dispositif de détection (9) est un dispositif de capture d'images disposé dans ou au niveau de soit le siège des toilettes (3), soit la cuvette (2) au-dessus d'une ligne d'eau (16).

10. Système de toilettes selon la revendication 9, dans lequel le premier dispositif de détection (9) est disposé de façon à être orienté vers le bas à un angle (α) situé entre 70 et 80 degrés, tel qu'un angle (α) de 75 degrés, avec l'horizontale (H) ou à un angle (β) situé entre 20 et 10 degrés, tel qu'un angle (β) de 15 degrés, avec la verticale (V), un angle de 90 degrés dans ce contexte correspondant à la verticale (V) ou à la direction de la gravité.

11. Procédé pour déterminer un type d'excrétion excrété dans une cuvette (2) d'un système de toilettes (1) selon l'une quelconque des revendications précédentes par un utilisateur du système de toilettes (1), ce procédé comprenant les étapes consistant à :
- utiliser au moins un premier dispositif de détection (9) du système de toilettes (1), capturant au moins un signal indicatif d'un type d'excrétion excrété dans la cuvette (2) par un utilisateur du système de toilettes (1),
- transmettre le signal capturé indicatif d'un type d'excrétion excrété dans le cuvette (2) par un utilisateur du système de toilettes (1) à un dispositif d'analyse (24) comprenant une unité de traitement de données (25) et une unité de stockage de données (26),
- recevoir, au moyen de l'unité de traitement de données (25), un ou plusieurs signaux transmis par l'au moins un premier dispositif de détection (9), et à
- analyser, au moyen de l'unité de traitement de données (25), le ou les signaux reçus transmis par l'au moins un premier dispositif de détection (9) afin de produire une sortie de données indicative du type d'excrétion excrété dans la cuvette (2) par un utilisateur du système de toilettes (1).

12. Procédé selon la revendication 11, et comprenant les étapes supplémentaires consistant à :
- utiliser au moins un deuxième dispositif de détection (10) du système de toilettes (2), capturant un signal indicatif d'une quantité d'excrétion excrétée dans la cuvette (1) par un utilisateur du système de toilettes (1),
- transmettre le signal capturé indicatif d'une quantité d'excrétion excrétée dans la cuvette (24) par un utilisateur du système de toilettes (1) au dispositif d'analyse (24),
- recevoir, au moyen de l'unité de traitement de données (25), un ou plusieurs signaux transmis par l'au moins un deuxième dispositif de détection (10), et à
- analyser, au moyen de l'unité de traitement de données (25), le ou les signaux reçus transmis par l'au moins un deuxième dispositif de détection (10) afin de produire une sortie de données indicative de la quantité d'excrétion excrétée dans la cuvette (2) par un utilisateur du système de toilettes (1),
et/ou
le dispositif d'analyse de données (24) comprenant en outre une unité de visualisation de données (27) et ce procédé comprenant en outre l'étape consistant à :
visualiser sur l'unité de visualisation de données (27), en utilisant l'unité de traitement de données (25), la sortie de données indicative du type d'excrétion excrété dans la cuvette (2) par un utilisateur du système de toilettes (1) et/ou la sortie de données indicative de la quantité d'excrétion excrétée dans la cuvette (2) par un utilisateur du système de toilettes (1).
